# FASCICULE DE BREVET EUROPEEN

(11) **EP 0 971 584 B1**
(45) Date de publication et mention de la délivrance du brevet: **22.01.2003**
(21) Numéro de dépôt: 98910821.2
(22) Date de dépôt: 24.02.1998
(51) Int. Cl.: A01N 37/16

(54) **COMPOSITION DESINFECTANTE A BASE D'ACIDE PERACETIQUE ET D'UN AGENT TENSIOACTIF NON IONIQUE**
DESINFEKTIONSMITTELZUSAMMENSETZUNG AUF DER BASIS VON PERESSIGSÄURE UND EINEM OBERFLÄCHENAKTIVEN STOFF
DISINFECTANT COMPOSITION WITH PERACETIC ACID AND NON-IONIC SURFACTANT BASE

(30) Priorité: 26.02.1997 FR 9702308
(43) Date de publication de la demande: 19.01.2000
(73) Titulaire: CHEMOXAL SA, F-75007 Paris (FR)
(72) Inventeur: HAMON, Catherine, F-92320 Châtillon (FR); TERAL, Gilles, F-75013 Paris (FR); GOUGES, Yves, F-75014 Paris (FR)
(74) Mandataire: Conan, Philippe Claude
(86) Numéro de dépôt international: FR9800360
(87) Numéro de publication internationale: WO98037762

(56) Documents cités:
- EP-A- 0 397 217
- EP-A- 0 596 493
- WO-A-94/14321
- DE-U- 9 312 055
- GB-A- 2 255 507

## Description

L'invention concerne de nouvelles compositions désinfectantes contenant de l'acide peracétique, du peroxyde d'hydrogène.

De telles solutions sont aujourd'hui largement utilisées dans de nombreux secteurs de l'industrie. L'intérêt croissant de l'acide peracétique réside dans sa large gamme d'activité biocide, en particulier vis-à-vis des bactéries, des algues, des levures, des moisissures, des champignons et des virus. Néanmoins, les solutions d'acide peracétique diluées présentent des performances de mouillage insuffisantes sur les surfaces couramment traitées, par exemple dans le domaine médical et hospitalier, dans les industries agro-alimentaires, pharmaceutiques, cosmétiques, micro-électroniques, les bio-industries, les collectivités et, de façon plus générale, dans toutes les activités pour lesquelles un contrôle de la population des micro-organismes est nécessaire. Les surfaces à traiter sont très diverses ; on peut citer par exemple, les locaux, les équipements, les matériels, les outils et ustensiles, les contenants, tels que les cuves, les emballages de conditionnement, les tuyaux. On a donc cherché à abaisser la tension superficielle de ces solutions désinfectantes par l'addition d'agents tensioactifs. Parmi les documents de l'état de la technique, on peut notamment citer ceux qui décrivent des compositions comportant un peracide et un agent tensioactif non ionique comme les demandes internationales de brevet publiées sous les numéros WO 88/08667, WO 93/10088 et WO 94/14321 ou les demandes de brevet européen EP 193 416 et EP 596 493.

Cependant, aucune des solutions désinfectantes à base d'acide peracétique décrites dans cet état de la technique, ne possède l'ensemble des propriétés nécessaires pour être commercialement acceptable ; ces propriétés sont les suivantes : la solution doit contenir à la fois l'acide peracétique et l'agent tensioactif, elle doit être stable dans le temps, elle doit posséder un fort pouvoir mouillant et un faible pouvoir moussant, elle doit être aisément rinçable, avoir un aspect limpide, elle doit contenir des quantités minimales de produits chimiques, tout en étant suffisamment active après dilution vis-à-vis des micro-organismes, et enfin le ou les agents tensioactifs utilisés doivent être compatibles avec l'acide peracétique.

La présente invention a donc pour objet une composition sous forme d'une solution aqueuse comprenant de 1 à 15% en poids d'acide peracétique, de 5 à 25% en poids de peroxyde d'hydrogène, de 5 à 25 % en poids d'acide acétique et de 0,1 à 1 % en poids d'un agent tensioactif non ionique, caractérisée en ce que :
a) ledit agent tensioactif a une concentration micellaire critique inférieure à 80mg/dm³ , mesurée dans de l'eau à 20°C ayant un degré de dureté égal à 25°TH; et,
b) le rapport pondéral entre ledit agent tensioactif et l'acide peracétique est inférieur à 0,2.

Dans ce qui précède et dans ce qui suit, la concentration micellaire critique (CMC) est, par définition, la concentration en solution de l'agent tensioactif au-dessus de laquelle une partie des molécules dispersées dans la solution se rassemble sous forme de micelles. La tension superficielle'est minimale à la CMC. La concentration micellaire critique est déterminée, sur des prises d'essais de 50 cm³ à l'aide d'un tensiomètre K12 commercialisé par KRUSS GmbH, en utilisant des éprouvettes de verre, dans de l'eau de ville à 20°C ayant un degré de dureté de 25°TH à cette température.

Il est bien entendu que la CMC est exprimée en poids de tensioactif à 100 %; dans le cas de produits commerciaux qui auraient des teneurs en agent tensioactif non ionique inférieures à 100%, il conviendra d'apporter les corrections nécessaires.

Lorsque l'on écrit que la composition selon l'invention comprend un agent tensioactif non ionique, cela signifie aussi que ladite composition peut comprendre un mélange de plusieurs agents tensioactifs non ioniques, étant entendu que dans ce cas, la CMC du mélange doit être inférieure à 80 mg/dm³.

Par agent tensioactif non ionique, on désigne notamment dans la présente invention, les composés comportant une chaîne hydrophile oxygénée. Parmi les agents de surface préférés disponibles dans le commerce, on peut citer notamment le Génapol 2822, le Génapol 2908, le Génapol 2908D, le Génapol 2909, le Triton DF 12, le Dowfax 20B102, le Akypo RO 90, le Synperonic LF RA 30, ou le Simulsol NW 90.

Dans une première variante préférée de la présente invention, l'agent tensioactif non ionique a une concentration micellaire critique inférieure à 50 mg/dm³. On peut citer comme agent tensioactif possédant une concentration micellaire critique inférieure à 50 mg/dm³, certains alcools gras alkoxylés seuls ou en mélange, tels que les alcools éthoxylés (OE), propoxylés (OP) ou éthoxylés et propoxylés (OE,OP) comme par exemple un mélange d'alcools gras alkoxylés en C₁₁, C₁₃, C₁₅ ayant un indice d'OE voisin de 6 à 7 et un indice d'OP voisin de 3 ou bien un mélange d'alcools gras alkoxylés en C₁₂ et C₁₄ ayant un indice d'OE voisin de 5 et un indice d'OP voisin de 3.

Dans une deuxième variante préférée de la présente invention, l'agent tensioactif non ionique est un éther carboxylique, ou acide carboxylique alkoxylé, et est de préférence un mélange d'éthers carboxyliques en C₁₆ et C₁₈ éthoxylés ayant un indice d'OE voisin de 9.

Les solutions aqueuses selon l'invention peuvent comporter en outre, les composés classiques connus de l'homme du métier tels que des stabilisants; de tels composés sont présents dans la solution d'acide peracétique et de peroxyde d'hydrogène utilisée pour la préparation des compositions selon l'invention; ce sont par exemple les acides forts, les agents séquestrants ou les capteurs de radicaux libres tels l'acide sulfurique, les acides phosphoniques ou encore l'acide dipicolinique ou les butyl hydroxy toluènes. Selon l'utilisation envisagée, la composition selon l'invention peut aussi contenir des colorants et/ou des parfums.

Dans une autre variante préférée de la présente invention, ledit agent tensioactif possède une concentration micellaire critique inférieure ou égale à 10mg/dm³ et, tout particulièrement, inférieure ou égale à 5mg/dm³.

Dans une autre variante préférée de la présente invention, la solution aqueuse comprend, de préférence, entre 2,5 % et 6 % en poids d'acide peracétique, entre 9 % et 20 % en poids de peroxyde d'hydrogène, entre 10 % et 20 % d'acide acétique et entre 0,10 % et 0,30 % en poids d'agent tensioactif non ionique.

Selon une autre variante préférée de la présente invention, la solution aqueuse comprend de 2,5 % à 5 % en poids d'acide peracétique, de 3 % à 15 % en poids de peroxyde d'hydrogéne, de 6% à 25% en poids d'acide acétique et de 0,1% à 1% en poids d'agent tensioactif non-ionique, ledit agent tensioactif non ionique ayant une concentration micellaire critique inférieure ou égale à 5mg/dm³, mesurée dans de l'eau à 20°C ayant un degré de dureté égal à 25° TH.

Comme il l'est illustré par les exemples suivants, les solutions selon l'invention sont stables, ont des propriétés mouillantes performantes, sont peu moussantes et sont facilement rinçables.

Ces solutions sont préparées selon les méthodes classiques de l'homme du métier à partir de solutions commerciales d'acide peracétique, de peroxyde d'hydrogène et de l'agent tensioactif pur ou en solution.

Dans un autre aspect de la présente invention, celle-ci a pour objet un procédé de préparation d'une solution par dilution dans l'eau d'une composition selon l'invention ainsi que la solution susceptible d'être obtenue par ladite dilution, caractérisé en ce que la concentration de l'agent tensioactif non ionique dans ladite solution diluée, est comprise entre 5 fois et 15 fois et, de préférence égale à environ 10 fois sa concentration micellaire critique mesurée dans de l'eau à 20°C ayant un degré de dureté égal à 25° TH. L'invention a particulièrement pour objet une solution susceptible d'être obtenue par dilution au 80^{e} dans l'eau, d'une composition selon l'invention comprenant entre 2,5 % et 6 % en poids d'acide peracétique et entre 10 % et 20 % en poids de peroxyde d'hydrogène, entre 10 % et 20 % d'acide acétique et entre 0,10 % et 0,30 % en poids d'agent tensioactif non ionique.

Dans une autre variante de la présente invention, on prépare une solution en diluant entre 40 et 300 fois, de préférence entre 80 et 240 fois dans l'eau, simultanément ou dans un ordre quelconque :
a) une composition sous forme de solution aqueuse comprenant de 0,1 % à 20 % en poids d'acide peracétique, de 2 % à 35 % en poids de peroxyde d'hydrogène, de 2 % à 45 % en poids d'acide acétique,
b) de 0,005 % à 2 % en poids d'un agent tensioactif non ionique, ledit agent tensioactif ayant une concentration micellaire critique inférieure ou égale à 80mg/dm³, en quantité telle que le rapport pondéral entre ledit agent tensioactif et l'acide peracétique est inférieur à 0,2.

Selon un autre aspect de la présente invention, celle-ci a pour objet un procédé de préparation d'une solution par dilution dans l'eau d'une composition telle que définie précédemment, caractérisé en ce que la concentration de l'agent tensioactif non ionique dans ladite solution diluée est comprise entre 1 et 2 fois la concentration micellaire critique mesurée dans de l'eau à 20°C ayant un degré de dureté égal à 25° TH.

L'invention a tout particulièrement pour objet une solution susceptible d'être obtenue par dilution entre 1500 fois et 3000 fois et, de préférence, environ 2500 fois, d'une composition telle que définie précédemment, comprenant de 2,5 % à 5 % en poids d'acide peracétique, de 3 % à 15 % en poids de peroxyde d'hydrogène, de 6% à 25% en poids d'acide acétique et de 0,1% à 1% en poids d'agent tensioactif non-ionique, ledit agent tensioactif non ionique ayant une concentration micellaire critique inférieure ou égale à 5mg/dm³, mesurée dans de l'eau à 20°C ayant un degré de dureté égal à 25° TH.

Selon l'utilisation qui en est faite, la composition, objet de la présente invention, est mise en oeuvre, soit non diluée, soit diluée en formant ainsi les solutions telles que décrites précédemment. C'est pourquoi, dans un autre aspect de la présente invention, celle-ci a pour objet l'utilisation de la composition et/ou des solutions telles que définies précédemment pour la désinfection des surfaces dures. Par surfaces dures, on entend par exemple les sols, les murs, les parois de récipients, les plateaux et les rayonnages, les surfaces de tout matériel, récipient ou équipement, utilisé par exemple dans les industries alimentaires et agro-alimentaires ou dans les activités de préparation, de transformation et conditionnement des aliments et des boissons, dans les bio-industries, les industries pharmaceutiques, les industries cosmétiques, les salles blanches, les serres, et les bâtiments de culture ou d'élevage ou encore dans le domaine de l'hygiène et de la santé; les compositions selon l'invention sont dans ce cas, utilisées pour désinfecter les locaux hospitaliers, les matériels médico-chirurgicaux, les matériels de dentisterie. Par surfaces dures, on entend par exemple soit des surfaces planés lisses soit des surfaces concaves lisses ou d'un aspect granuleux ou même des surfaces d'objets de formes complexes comportant en particulier des cavités telles que les tuyaux ou les cathéters.

La surface à traiter peut être en tout matériau et notamment en verre, en métal, par exemple en acier émaillé ou en acier inoxydable, en céramique, en polymère organique tel que par exemple les polyéthylènes, les polypropylènes, les polycarbonates, les polyamides, les polyesters tels que les polyéthylènes téréphtalates (PET), les polyuréthanes, les polymères fluorés tels que les PTFE, PVDF, PFA, les polychlorures de vinyle (PVC).

Dans un aspect préféré de la présente invention, les solutions telles que définies précédemment sont utilisées pour nettoyer les récipients utilisés dans l'industrie alimentaire et destinés à être en contact avec des produits alimentaires et, notamment, avec le lait ou les produits dérivés du lait.

Dans un autre aspect préféré, les solutions telles que décrites précédemment sont utilisées pour la désinfection des bouteilles ou de tout récipient destiné à recevoir des liquides alimentaires comme les boissons, telles que par exemple les eaux minérales, les jus de fruits, les bières, dans l'industrie d'embouteillage et, notamment, le nettoyage des bouteilles en polyéthylène téréphtalate (PET).

Dans un autre aspect de la présente invention, les compositions et/ou solutions telles que définies précédemment sont utilisées en hygiène hospitalière, notamment pour désinfecter le matériel médico-chirurgical.

Selon un autre aspect de la présente invention, la composition selon l'invention est utilisée dans le traitement de désinfection des solutions nutritives de cultures ornementales (plantes en pots).

La composition sous forme d'une solution aqueuse comprenant de 2,5 % à 5 % en poids d'acide peracétique, de 3 % à 15 % en poids de peroxyde d'hydrogène, de 6% à 25% en poids d'acide acétique et de 0,1% à 1% en poids d'agent tensioactif non-ionique, ledit agent tensioactif non ionique ayant une concentration micellaire critique inférieure ou égale à 5mg/dm³, mesurée dans de l'eau à 20°C ayant un degré de dureté égal à 25° TH est particulièrement appropriée à cette application ; elle est mise en oeuvre par dilution entre 1500 fois et 3000 fois et de préférence 2500 fois dans la solution nutritive des cultures florales, qui est recyclée et réutilisée, de manière à maintenir dans la solution nutritive recirculant une concentration pondérale en acide peracétique autour de 10⁻³ % (10 ppm). A cette concentration en acide peracétique, l'activité microbicide et algicide est suffisante et la concentration en peroxyde d'hydrogène est suffisamment faible pour ne pas favoriser par libération d'oxygène la croissance des algues et empêcher l'action du peracide.

De par leur stabilité et leur innocuité, les compositions et/ou solutions telles que définies précédemment peuvent aussi être utilisées en milieu non hospitalier notamment, par le praticien en médecine libérale pour le nettoyage et la désinfection de son matériel de consultation.

Dans un dernier aspect de la présente invention, la composition ou la solution diluée est absorbée sur un matériau poreux tel qu'une éponge, du papier, ou du tissu, pour former une formulation prête à l'emploi sur support solide telle que, par exemple, une lingette.

Les exemples suivants illustrent l'invention sans toutefois la limiter.

### A - PREPARATION DES COMPOSITIONS SELON L'INVENTION

| **Composition** | **APA % W/W** | **H**_{**2**}**O**_{**2**} **% W/W** | **AcOH% W/W** | **agent tensioactif % W/W** |
|---|---|---|---|---|
| 1 | 5,5 | 14,5 | 16,5 | Génapol 2908D 0,24 |
| 2 | 2,5 | 15,0 | 7,0 | Génapol 2908D 0,12 |
| 3 | 2,5 | 18,5 | 5,0 | Génapol 2909 0,16 |
| 4 | 3,5 | 13,0 | 12,0 | Génapol 2908D 0,12 |
| 5 | 3,5 | 18,0 | 7,5 | Génapol 2909 0,16 |
| APA : acide peracétique | | | | |
| H₂O₂ : peroxyde d'hydrogène | | | | |
| AcOH : acide acétique. | | | | |

Le Génapol 2908D et le Génapol 2909 sont des agents tensioactifs non ioniques, commercialisés par la Société HÖCHST.

La concentration micellaire critique du Génapol 2908D, mesurée dans de l'eau de ville à 20°C, ayant un degré de dureté de 25 TH, est égale à 5mg/dm³.

La concentration micellaire critique du Génapol 2909, mesurée dans les mêmes conditions, est égale à 7mg/dm³.

A titre de comparaison, le Triton DF16 qui est l'agent tensioactif utilisé dans l'expérience décrite dans la demande internationale de brevet WO 94/14321 a une concentration micellaire critique supérieure à 500 mg/dm³.

Les compositions selon l'invention ont été préparées selon les méthodes classiques de l'homme du métier par mélanges successifs des constituants.

### B - ETUDE DE LA STABILITE DES COMPOSITIONS SELON L'INVENTION.

On a mesuré la perte d'oxygène actif des compositions 1 à 5 après 13 mois de stockage au laboratoire.

Pour chacune de ces compositions, il a été observé que la perte d'oxygène actif était inférieure à 5 % en valeur relative, au bout de cette période.

### C - ETUDE DE LA MOUILLABILITE DES COMPOSITIONS SELON L'INVENTION

I) La mouillabilité des compositions selon l'invention, diluées 40 ou 80 fois ont été analysées en utilisant la méthode de WILHELMY pour la mesure des angles de contact et la méthode de la lame pour la mesure de la tension superficielle. Les mesures sont effectuées avec un tensiomètre K12 sur parafilm, à 20°C.
On obtient les résultats suivants :

| Composition | γ^{L} (mN/m) | θₐ^{(°)} |
|---|---|---|
| 1 dilution : 80 fois | ∼ 30 | ∼ 57 |
| 2 dilution : 40 fois | ∼ 30 | ∼ 57 |
| 3 dilution : 40 fois | ∼ 30,5 | ∼ 59 |
| 4 dilution :40 fois | ∼ 26,5 | ∼ 59 |
| 5 dilution : 40 fois | ∼ 31 | ∼ 68 |
| θₐ : angle de contact à l'avancée | | |
| γ^{L} : tension de surface totale | | |

les résultats obtenus démontrent les bonnes caractéristiques de mouillabilité des compositions selon l'invention.
II) A titre de comparaison avec une composition selon l'état de la technique, on a mesuré à 20°C et à 50°C, dans des conditions identiques, les tensions superficielles totales γ^{L} ainsi que les angles de contact à l'avancée θ°ₐ , sur parafilm et sur PET du bain désinfectant obtenu par dilution au 80° de la composition n° 1, avec ceux d'un bain désinfectant obtenu par dilution au 80^{e} de la composition A comprenant 5,5 % d'acide peracétique, 14,5 % de peroxyde d'hydrogène, 16,5 % d'acide acétique et 0,24 % de l'agent tensioactif non ionique Triton DF 16.

Les résultats sont les suivants :
**a)** T = 20°C

| | γ^{L}(mN/m) | θ°ₐ | Matériau |
|---|---|---|---|
| composition 1 | 32,0 | 73,3 | parafilm |
| | 32,0 | 57,6 | PET |
| Composition A | 49,4 | 96,3 | Parafilm |
| | 49,4 | 86,8 | PET |

**b)** T = 50°C

| | γ^{L}(mN/m) | θ°ₐ | Matériau |
|---|---|---|---|
| composition 1 | 28,5 | 62,9 | parafilm |
| | 28,5 | 32,4 | PET |
| Composition A | 42,5 | 81,0 | Parafilm |
| | 42,5 | 70,0 | PET |

Ces résultats démontrent que quels que soient la température et le matériau concerné, la composition 1 selon l'invention, présente de meilleures caractéristiques de mouillabilité que la composition A.

### D - ETUDE DE LA RINCABILITE DES COMPOSITIONS SELON L'INVENTION.

Le rinçage de la composition n° 1, diluée 80 fois a été examiné dans une éprouvette en PET avec un tensiomètre K12 en utilisant la méthode de WILHELMY pour la mesure des angles de contact et la méthode de la lame pour la mesure de la tension superficielle.

Après chaque rinçage, on a mesuré la tension de surface γ^{L} de l'eau de rinçage.

L'essai a montré que la composition n° 1 selon l'invention se rinçait mieux dans les mêmes conditions opératoires, qu'une composition comprenant de l'acide peracétique, du peroxyde d'hydrogène et de l'acide acétique dans les mêmes proportions mais comportant comme agent tensioactif le Texapon P3 Spéciale, qui a une CMC égale à 80 à 20°C dans l'eau de ville à 25 TH de dureté.

### E - ETUDE DU POUVOIR MOUSSANT DES COMPOSITIONS SELON L'INVENTION.

La composition n° 1, diluée 80 fois, et les compositions n°2 et n° 4, chacune diluée 40 fois, sont préchauffées à 50°C, puis leur pouvoir moussant est mesuré selon la norme AFNOR NF T73-404. On obtient les résultats suivants :

| **composition** | **Temps (secondes)** | **Hauteur de mousse (mm)** |
|---|---|---|
| N° 1 | 0 | 20 |
| | 60 | 10 |
| | 300 | 5 |
| N° 2 | 0 | 10 |
| | 60 | 10 |
| | 300 | 5 |
| N° 3 | 0 | 10 |
| | 60 | 10 |
| | 300 | 5 |

Ces résultats démontrent les propriétés peu moussantes des compositions selon l'invention.

### F - PROPRIETES BIOCIDES

L'efficacité fongicide a été étudiée sur Candida parapsilosis selon la norme NFT 72201 à 20°C ; on a constaté un abattement logarithmique de 5 de la population de fongi après 15 minutes de contact avec la composition n° 1 selon l'invention, diluée 80 fois dans l'eau pour atteindre la concentration initiale d'acide peracétique égale à 3000 ppm (parties par million).

Lorsque, selon la même norme NFT 72201, on analyse l'efficacité fongicide d'une solution ayant les mêmes concentrations en acide peracétique, en peroxyde d'hydrogène et en acide acétique, que la composition 1, mais ne contenant pas d'agent tensioactif, on observe que l'abattement logarithmique de 5 de la population de fongi se produit en 15 minutes si la dilution de ladite solution conduit à une concentration en acide peracétique au moins égale à 3300 ppm, ce qui montre qu'il existe de façon inattendue, une synergie en milieu acide à pH inférieur à 4 entre l'acide peracétique et l'agent tensioactif non ionique selon l'invention.

### G - CONCENTRATION MICELLAIRE CRITIQUE (CMC) D'AGENTS TENSIOACTIFS NON IONIQUES.

La concentration micellaire critique d'agents tensioactifs non-ioniques commerciaux est déterminée, sur des prises d'essais de 50 cm³ à l'aide d'un tensiomètre K12 commercialisé par KRUSS GmbH, en utilisant des éprouvettes de verre, dans de l'eau de ville à 20°C ayant un degré de dureté de 25°TH à cette température.

Il est bien entendu que la CMC est exprimée en poids de tensioactif à 100 %; dans le cas de produits commerciaux qui auraient des teneurs en agent tensioactif non ionique inférieures à 100%, il conviendra d'apporter les corrections nécessaires.

On obtient les résultats suivants :
**1) Tensioactifs mis en oeuvre dans la présente invention :**

| **nom** | **Composition chimique du tensioactif** | **CMC mg/dm**^{**3**} |
|---|---|---|
| Génapol® 2822 | mélange d'alcools alkoxylés en C₁₀, C₁₂, C₁₄ (5 OE, 4 OP) | 38 |
| Génapol® 2908D | mélange d'alcools alkoxylés en C₁₁, C₁₃, C₁₅ (6 à 7 OE, 3 OP) | 5 |
| Génapol® 2909 | mélange d'alcools alkoxylés en C₁₂ et C₁₄ (5 OE, 3 OP) | 7 |
| Simulsol® NW 900 | alcools alkoxylés | 79 |
| Triton® DF12 | Ether benzylique d'alcools alkoxylés en C₈, C₁₀ (2 OE, 5 OP) | 28 |
| Triton® CF 10 | Ether benzylique d'alcool éthoxylés en C₈ (16 OE) | 69 |
| Akypo® RO 90 | Ether carboxylique éthoxylé en C₁₆, C₁₈ (9 OE) | 2 |
| Dowfax® 20B102 | | 7 |

**2) Tensioactifs ayant une CMC ≥ 80**

| **nom** | **Composition chimique du tensioactif** | **CMC mg/dm**^{**3**} |
|---|---|---|
| Texapon P3 Spécial | monyl phénol éthoxylé (60 E) | 80 |
| Triton DF16 | mélange d'alcools alkoxylés en C₈, C₁₀ (6 OE, 3 OP) | 515 |
| Akypo LF4 | Ether carboxylique éthoxylé en C₆, C₈ (7 OE) | 412 |
| Akypo LF2 | Ether carboxylique éthoxylé en C₈ (8 OE) | 84 |

## Revendications

1. Composition sous forme d'une solution aqueuse comprenant de 1 % à 15 % en poids d'acide peracétique, de 5 % à 25 % en poids de peroxyde d'hydrogène, de 5 % à 25 % en poids d'acide acétique et de 0,1 % à 1 % en poids d'un agent tensioactif non ionique, **caractérisée en ce que** :
a) ledit agent tensioactif a une concentration micellaire critique inférieure à 80mg/dm³, mesurée à 20°C dans une eau ayant un degré de dureté égal à 25° TH ; et,
b) le rapport pondéral entre ledit agent tensioactif et l'acide peracétique est inférieur à 0,2.

2. Composition telle que définie à la revendication 1 dans laquelle l'agent tensioactif non ionique a une concentration micellaire critique inférieure ou égale à 50 mg/dm³.

3. Composition telle que définie à la revendication 2 dans laquelle l'agent tensioactif non ionique est un alcool gras alkoxylé ou un mélange d'alcools gras alkoxylés et, de préférence, un mélange d'alcools gras alkoxylés en C₁₁, C₁₃, C₁₅ ayant un indice d'OE voisin de 6 ou de 7 et un indice d'OP voisin de 3 ou bien un mélange d'alcools gras alkoxylés en C₁₂ et C₁₄ ayant un indice d'OE voisin de 5 et un indice d'OP voisin de 3.

4. Composition telle que définie à la revendication 2 dans laquelle l'agent tensioactif non ionique est un éther carboxylique et de préférence un mélange d'éthers carboxyliques éthoxylés en C₁₆ et C₁₈ ayant un indice d'OEB voisin à 9.

5. Composition telle que définie à l'une des revendications 1 à 4 dans laquelle ledit agent tensioactif possède une concentration micellaire critique inférieure ou égale à 10 mg/dm³ et tout particulièrement inférieure ou égale à 5mg/dm³.

6. Composition telle que définie à l'une des revendications 4 ou 5 dans laquelle la solution aqueuse comprend entre 2,5 % et 6 % en poids d'acide peracétique et entre 10 % et 20% en poids de peroxyde d'hydrogène, entre 10 % et 20 % d'acide acétique et entre 0,10 % et 0,30 % en poids d'un agent tensioactif non ionique.

7. Composition sous forme d'une solution aqueuse comprenant de 2,5 % à 5 % en poids d'acide peracétique, de 3 % à 15 % en poids de peroxyde d'hydrogène, de 6% à 25% en poids d'acide acétique et de 0,1% à 1% en poids d'agent tensioactif non-ionique, ledit agent tensioactif non ionique ayant une concentration micellaire critique inférieure ou égale à 5mg/dm³, mesurée dans de l'eau à 20°C ayant un degré de dureté égal à 25° TH, et le rapport pondéral entre ledit agent tensioactif et l'acide peracétique est inférieur à 0,2.

8. Solution susceptible d'être obtenue par dilution dans l'eau de la composition telle que définie à l'une des revendications 1 à 7, **caractérisée en ce que** la concentration dudit agent tensioactif non ionique dans la solution est comprise entre 5 fois et 15 fois et de préférence égale à environ 10 fois sa concentration micellaire critique, mesurée dans de l'eau à 20°C ayant un degré de dureté égal à 25°TH.

9. Solution susceptible d'être obtenue par dilution au 80^{ème} dans l'eau d'une composition telle que définie à la revendication 7, **caractérisée en ce que** la concentration dudit agent tensioactif non ionique dans ladite solution est comprise entre 5 fois et 10 fois sa concentration micellaire critique, mesurée dans de l'eau à 20°C ayant un degré de dureté égal à 25°TH.

10. Procédé de préparation d'une solution, **caractérisé en ce que** l'on dilue entre 40 et 300 fois, de préférence entre 80 et 240 fois, dans l'eau, simultanément ou dans un ordre quelconque :
a) une composition sous forme d'une solution aqueuse comprenant de 0,1 % à 20 % en poids d'acide peracétique, de 2 % à 35 % en poids de peroxyde d'hydrogène, de 2 % à 45 % en poids d'acide acétique,
b) de 0,005 % à 2 % en poids d'un agent tensioactif non ionique, ledit agent tensioactif ayant une concentration micellaire critique inférieure ou égale à 80mg/dm³; en quantité telle que le rapport pondéral entre ledit agent tensioactif et l'acide peracétique est inférieur à 0,2.

11. Procédé de préparation d'une solution, **caractérisé en ce que** l'on dilue entre 1500 fois et 3000 fois et de préférence environ 2500 fois, dans l'eau, la composition telle que définie à la revendication 7.

12. Solution susceptible d'être obtenue par dilution dans l'eau de la composition telle que définie à l'une des revendications 1 à 7, **caractérisé en ce que** la concentration de l'agent tensioactif non ionique dans ladite solution diluée est comprise entre 1 et 2 fois la concentration micellaire critique mesurée dans de l'eau à 20°C ayant un degré de dureté égal à 25° TH.

13. Solution susceptible d'être obtenue par dilution environ 2500 fois dans l'eau de la composition telle que définie à la revendication 7.

14. Utilisation des compositions telles que définies à l'une des revendications 1 à 7 pour désinfecter les surfaces dures.

15. Utilisation des solutions telles que définies à l'une des revendications 8 à 13 pour désinfecter les surfaces dures.

16. Utilisation selon l'une des revendications 14 ou 15 pour laquelle la surface à désinfecter est en verre, en métal, en acier émaillé, en acier inoxydable, en céramique, en polymère organique tel que par exemple les polyéthylènes, les polypropylènes, les polycarbonates, les polyamides, les polyesters tels que les polyéthylènes téréphtalates (PET), les polyuréthanes, les polymères fluorés tels que les PTFE, PVDF, PFA, les polychlorures de vinyle (PVC).

17. Utilisation selon l'une des revendications 14 à 16, pour désinfecter les récipients utilisés dans l'industrie alimentaire et destinés à être en contact avec des produits alimentaires et notamment avec le lait ou les produits dérivés du lait.

18. Utilisation selon l'une des revendications 14 à 16, pour désinfecter les bouteilles ou tout récipient destiné à recevoir des boissons, dans l'industrie d'embouteillage.

19. Utilisation selon la revendication 18, pour laquelle les bouteilles sont en polyéthylènétéréphtalate.

20. Utilisation de la composition telle que définie à la revendication 7 ou de la solution telle que définie à l'une des revendications 11 à 13, dans le traitement de désinfection des solutions nutritives des cultures ornementales.

21. Procédé de désinfection en continu de la solution nutritive de cultures florales en pots, **caractérisé en ce que** l'on maintient dans ladite solution, une concentration en acide peracétique autour de 10⁻³ % en poids, par introduction dans le circuit de recirculation de la composition telle que définie à la revendication 8 à un débit permettant une dilution environ au 2500^{ème} de ladite composition dans ladite solution nutritive.

22. Utilisation selon l'une des revendications 14 ou 15 en hygiène hospitalière, notamment pour désinfecter le matériel médico-chirurgical.

23. Utilisation selon l'une des revendications 14 ou 15, en milieu non hospitalier, pour désinfecter le matériel de consultation du praticien de médecine libérale.

24. Formulation prête à l'emploi sur support solide, **caractérisée en ce qu'**elle comprend une composition telle que définie à l'une des revendications 1 à 7, absorbée sur un matériau poreux tel que de l'éponge, du papier ou du tissu.

25. Formulation prête à l'emploi sur support solide, **caractérisée en ce qu'**elle comprend une solution telle que définie à l'une des revendications 8 à 13, absorbée sur un matériau poreux tel que de l'éponge, du papier ou du tissu.

## Patentansprüche

1. Zusammensetzung in Form einer wäßrigen Lösung, enthaltend 1 bis 15 Gew.-% Peressigsäure, 5 bis 25 Gew.-% Wasserstoffperoxid, 5 bis 25 Gew.-% Essigsäure und 0,1 bis 1 Gew.-% eines nichtionischen Tensids, **dadurch gekennzeichnet, daß**:
a) das Tensid eine kritische Micellenkonzentration von weniger als 80 mg/dm³, gemessen bei 20°C in Wasser mit einem Härtegrad von 25° GH, aufweist und
b) das Gewichtsverhältnis zwischen dem Tensid und der Peressigsäure kleiner als 0,2 ist.

2. Zusammensetzung nach Anspruch 1, worin das nichtionische Tensid eine kritische Micellenkonzentration von kleiner gleich 50 mg/dm³ aufweist.

3. Zusammensetzung nach Anspruch 2, worin es sich bei dem nichtionischen Tensid um ein Fettalkoholalkoxylat oder ein Gemisch von Fettalkoholalkoxylaten und vorzugsweise ein Gemisch von C₁₁-, C₁₃- und C₁₅-Fettalkoholalkoxylaten mit einem EO-Index von etwa 6 oder 7 und einem PO-Index von etwa 3 oder ein Gemisch von C12- und C₁₄-Fettalkoholalkoxylaten mit einem EO-Index von etwa 5 und einem PO-Index von etwa 3 handelt.

4. Zusammensetzung nach Anspruch 2, worin es sich bei dem nichtionischen Tensid um einen Carbonsäureether und vorzugsweise ein Gemisch von ethoxylierten C₁₆- und C₁₈-Carbonsäureethern mit einem EO-Index von etwa 9 handelt.

5. Zusammensetzung nach einem der Ansprüche 1 bis 4, worin das Tensid eine kritische Micellenkonzentration kleiner gleich 10 mg/dm³ und insbesondere kleiner gleich 5 mg/dm³ aufweist.

6. Zusammensetzung nach Anspruch 4 oder 5, worin die wäßrige Lösung zwischen 2,5 und 6 Gew.-% Peressigsäure und zwischen 10 und 20 Gew.-% Wasserstoffperoxid, zwischen 10 und 20 Gew.-% Essigsäure und zwischen 0,10 und 0,30 Gew.-% eines nichtionischen Tensids enthält.

7. Zusammensetzung in Form einer wäßrigen Lösung, enthaltend 2,5 bis 5 Gew.-% Peressigsäure, 3 bis 15 Gew.-% Wasserstoffperoxid, 6 bis 25 Gew.-% Essigsäure und 0,1 bis 1 Gew.-% eines nichtionischen Tensids, wobei das nichtionische Tensid eine kritische Micellenkonzentration kleiner gleich 5 mg/dm³, gemessen in Wasser bei 20°C mit einem Härtegrad von 25° GH, aufweist und das Gewichtsverhältnis zwischen dem Tensid und der Peressigsäure kleiner als 0,2 ist.

8. Durch Verdünnen der Zusammensetzung nach einem der Ansprüche 1 bis 7 mit Wasser erhältliche Lösung, **dadurch gekennzeichnet, daß** die Konzentration des nichtionischen Tensids in der Lösung zwischen dem 5- und 15fachen seiner kritischen Micellenkonzentration, gemessen in Wasser bei 20°C mit einem Härtegrad von 25° GH, liegt und vorzugsweise ungefähr gleich dem 10fachen seiner kritischen Micellenkonzentration, gemessen in Wasser bei 20°C mit einem Härtegrad von 25° GH, ist.

9. Durch 80faches Verdünnen einer Zusammensetzung nach Anspruch 7 mit Wasser erhältliche Lösung, **dadurch gekennzeichnet, daß** die Konzentration des nichtionischen Tensids in der Lösung zwischen dem 5- und 10fachen seiner kritischen Micellenkonzentration, gemessen in Wasser bei 20°C mit einem Härtegrad von 25° GH, liegt.

10. Verfahren zur Herstellung einer Lösung, **dadurch gekennzeichnet, daß** man:
a) eine Zusammensetzung in Form einer wäßrigen Lösung, enthaltend 0,1 bis 20 Gew.-% Peressigsäure, 2 bis 35 Gew.-% Wasserstoffperoxid und 2 bis 45 Gew.-% Essigsäure und
b) 0,005 bis 2 Gew.-% eines nichtionischen Tensids mit einer kritischen Micellenkonzentration kleiner gleich 80 mg/dm³ in einer solchen Menge, daß das Gewichtsverhältnis zwischen dem Tensid und der Peressigsäure kleiner als 0,2 ist,
gleichzeitig oder in beliebiger Reihenfolge mit Wasser zwischen 40- und 300fach, vorzugsweise zwischen 80- und 240fach, verdünnt.

11. Verfahren zur Herstellung einer Lösung, **dadurch gekennzeichnet, daß** man die Zusammensetzung nach Anspruch 7 mit Wasser zwischen 1500- und 3000fach und vorzugsweise ungefähr 2500fach verdünnt.

12. Durch Verdünnen der Zusammensetzung nach einem der Ansprüche 1 bis 7 mit Wasser erhältliche Lösung, **dadurch gekennzeichnet, daß** die Konzentration des nichtionischen Tensids in der verdünnten Lösung zwischen dem 1- und 2fachen der kritischen Micellenkonzentration, gemessen in Wasser bei 20°C mit einem Härtegrad von 25° GH, liegt.

13. Durch ungefähr 2500faches Verdünnen der Zusammensetzung nach Anspruch 7 mit Wasser erhältliche Lösung.

14. Verwendung von Zusammensetzungen nach einem der Ansprüche 1 bis 7 zur Desinfektion von harten Oberflächen.

15. Verwendung von Lösungen nach einem der Ansprüche 8 bis 13 zur Desinfektion von harten Oberflächen.

16. Verwendung nach Anspruch 14 oder 15, bei der die zu desinfizierende Oberfläche aus Glas, Metall, emailliertem Stahl, rostfreiem Stahl, Keramik oder organischem Polymer, wie beispielsweise Polyethylen, Polypropylen, Polycarbonat, Polyamid, Polyester, wie Polyethylenterephthalat (PET), Polyurethan, Fluorpolymeren, wie PTFE, PVDF und PFA, und Polyvinylchlorid (PVC) besteht.

17. Verwendung nach einem der Ansprüche 14 bis 16 zur Desinfektion von Behältern, die in der Lebensmittelindustrie verwendet werden und für den Kontakt mit Lebensmittelprodukten und insbesondere mit Milch oder Milchprodukten vorgesehen sind.

18. Verwendung nach einem der Ansprüche 14 bis 16 zur Desinfektion von Flaschen oder Behältern zur Aufnahme von Getränken in der Flaschenabfüllindustrie.

19. Verwendung nach Anspruch 18, bei der die Flaschen aus Polyethylenterephthalat bestehen.

20. Verwendung der Zusammensetzung nach Anspruch 7 oder der Lösung nach einem der Ansprüche 11 bis 13 bei der Desinfektionsbehandlung von Nährlösungen für Ornamentalkulturen.

21. Verfahren zur kontinuierlichen Desinfektion der Nährlösung für Blumenkulturen in Töpfen, **dadurch gekennzeichnet, daß** man in der Lösung eine Peressigsäurekonzentration von ungefähr 10⁻³ Gew.-% aufrechterhält, indem man in den Rezirkulationskreislauf die Zusammensetzung nach Anspruch 8 mit einer solchen Strömungsrate einträgt, daß sich eine etwa 2500fache Verdünnung der Zusammensetzung in der Nährlösung ergibt.

22. Verwendung nach Anspruch 14 oder 15 in der Krankenhaushygiene, insbesondere zur Desinfektion von medizinisch-chirurgischen Gerätschaften.

23. Verwendung nach Anspruch 14 oder 15 in Nichtkrankenhausumgebung, insbesondere zur Desinfektion von Gerätschaften für die Konsultation freier Ärzte.

24. Gebrauchsfertige Formulierung auf einem festen Träger, **dadurch gekennzeichnet, daß** sie eine von einem porösen Material, wie einem Schwamm, Papier oder Stoff, absorbierte Zusammensetzung nach einem der Ansprüche 1 bis 7 enthält.

25. Gebrauchsfertige Formulierung auf einem festen Träger, **dadurch gekennzeichnet, daß** sie eine von einem porösen Material, wie einem Schwamm, Papier oder Stoff, absorbierte Lösung nach einem der Ansprüche 8 bis 13 enthält.

## Claims

1. Composition in the form of an aqueous solution comprising from 1% to 15% by weight of peracetic acid, from 5% to 25% by weight of hydrogen peroxide, from 5% to 25% by weight of acetic acid and from 0.1% to 1% by weight of a nonionic surfactant, **characterized in that**:
a) the said surfactant has a critical micelle concentration of less than 80 mg/dm³, measured at 20°C in water having a degree of hardness equal to 25° TH; and,
b) the weight ratio between the said surfactant and the peracetic acid is less than 0.2.

2. Composition as defined in Claim 1, in which the nonionic surfactant has a critical micelle concentration of less than or equal to 50 mg/dm³.

3. Composition as defined in Claim 2, in which the nonionic surfactant is an alkoxylated fatty alcohol or a mixture of alkoxylated fatty alcohols and, preferably, a mixture of C₁₁, C₁₃. and C₁₅ alkoxylated fatty alcohols having an EO value in the region of 6 or 7 and a PO value in the region of 3 or alternatively a mixture of C₁₂ and C₁₄ alkoxylated fatty alcohols having an EO value in the region of 5 and a PO value in the region of 3.

4. Composition as defined in Claim 2, in which the nonionic surfactant is a carboxylic ether, and preferably a mixture of C₁₆ and C₁₈ ethoxylated carboxylic ethers having an EO value in the region of 9.

5. Composition as defined in one of Claims 1 to 4, in which the said surfactant has a critical micelle concentration of less than or equal to 10 mg/dm³ and most particularly less than or equal to 5 mg/dm³.

6. Composition as defined in either of Claims 4 and 5, in which the aqueous solution comprises between 2.5% and 6% by weight of peracetic acid and between 10% and 20% by weight of hydrogen peroxide, between 10% and 20% of acetic acid and between 0.10% and 0.30% by weight of a nonionic surfactant.

7. Composition in the form of an aqueous solution comprising from 2.5% to 5% by weight of peracetic acid, from 3% to 15% by weight of hydrogen peroxide, from 6% to 25% by weight of acetic acid and from 0.1% to 1% by weight of nonionic surfactant, the said nonionic surfactant having a critical micelle concentration of less than or equal to 5 mg/dm³, measured in water at 20°C having a degree of hardness equal to 25° TH, and the weight ratio between the said surfactant and the peracetic acid is less than 0.2.

8. Solution which can be obtained by diluting in water the composition as defined in one of Claims 1 to 7, **characterized in that** the concentration of the said nonionic surfactant in the solution is between 5 times and 15 times, and preferably is equal to about 10 times its critical micelle concentration, measured in water at 20°C having a degree of hardness equal to 25° TH.

9. Solution which can be obtained by diluting 1/80 in water a composition as defined in Claim 7, **characterized in that** the concentration of the said nonionic surfactant in the said solution is between 5 times and 10 times its critical micelle concentration, measured in water at 20°C having a degree of hardness equal to 25° TH.

10. Method for preparing a solution, **characterized in that** the following are diluted between 40 and 300-fold, preferably between 80 and 240-fold, in water, simultaneously or in any order:
a) a composition in the form of an aqueous solution comprising from 0.1% to 20% by weight of peracetic acid, from 2% to 35% by weight of hydrogen peroxide, from 2% to 45% by weight of acetic acid,
b) from 0.005% to 2% by weight of a nonionic surfactant, the said surfactant having a critical micelle concentration of less than or equal to 80 mg/dm³; in a quantity such that the weight ratio between the said surfactant and the peracetic acid is less than 0.2.

11. Method for preparing a solution, **characterized in that** the composition as defined in Claim 7 is diluted between 1 500-fold and 3 000-fold and preferably about 2 500-fold in water.

12. Solution which can be obtained by diluting in water the composition as defined in one of Claims 1 to 7, **characterized in that** the concentration of nonionic surfactant in the said dilute solution is between 1 and 2 times the critical micelle concentration measured in water at 20°C having a degree of hardness equal to 25° TH.

13. Solution which can be obtained by diluting about 2 500-fold in water the composition as defined in Claim 7.

14. Use of the compositions as defined in one of Claims 1 to 7 for disinfecting hard surfaces.

15. Use of the solutions as defined in one of Claims 8 to 13 for disinfecting hard surfaces.

16. Use according to either of Claims 14 and 15, for which the surface to be disinfected is made of glass, metal, enamelled steel, stainless steel, ceramic, an organic polymer such as for example polyethylenes, polypropylenes, polycarbonates, polyamides, polyesters such as polyethylene terephthalates (PET), polyurethanes, fluorinated polymers such as PTFE, PVDF, PFA, polyvinyl chlorides (PVC).

17. Use according to one of Claims 14 to 16, for disinfecting the containers used in the food industry and intended to be in contact with food products and in particular with milk or products derived from milk.

18. Use according to one of Claims 14 to 16, for disinfecting bottles or any container intended to receive drinks, in the bottling industry.

19. Use according to Claim 18, for which the bottles are made of polyethylene terephthalate.

20. Use of the composition as defined in Claim 7 or of the solution as defined in one of Claims 11 to 13, in the disinfection of the nutritive solutions of ornamental crops.

21. Method for the continuous disinfection of the nutritive solution of floral crops in pots, **characterized in that** a peracetic acid solution of around 10⁻³% by weight is maintained in the said solution by introducing into the circuit for recirculating the composition as defined in Claim 8 at a flow rate allowing an approximately 1/2 500 dilution of the said composition in the said nutritive solution.

22. Use according to either of Claims 14 and 15 in hospital hygiene, in particular for disinfecting medical and surgical equipment.

23. Use according to either of Claims 14 and 15, in a non-hospital setting, for disinfecting the consultation equipment used by the practitioner of liberal medicine.

24. Ready-to-use formulation on a solid support, **characterized in that** it comprises a composition as defined in one of Claims 1 to 7, absorbed on a porous material such as sponge, paper or cloth.

25. Ready-to-use formulation on a solid support, **characterized in that** it comprises a solution as defined in one of Claims 8 to 13, absorbed on a porous material such as sponge, paper or cloth.
